# EUROPEAN PATENT SPECIFICATION

(11) **EP 1 331 958 B1**
(45) Date of publication and mention of the grant of the patent: **08.09.2004**
(21) Application number: 02704635.8
(22) Date of filing: 15.03.2002
(51) Int. Cl.: A61M 5/32, A61B 17/34

(54) **NEEDLE CANNULA**
NADEL CANNULA
CANULE

(30) Priority: 23.03.2001 DK 200100483
(43) Date of publication of application: 06.08.2003
(60) Divisional application: 04012277.2
(73) Proprietor: NOVO NORDISK A/S, 2880 Bagsvaerd (DK); Nipro Corporation, Osaka 531 (JP)
(72) Inventor: KLINT, Henrik, Sonderskov, DK-2880 Lyngby (DK); HIGAKI, Yoshio, Gunma, Gunma 374-0031 (JP)
(74) Representative: Kellberg, Lars
(86) International application number: PCT/DK2002/000175
(87) International publication number: WO 2002/076540

(56) References cited:
- DE-A1- 2 408 852
- DE-A1- 4 109 442
- US-A- 4 405 314
- US-A- 5 002 535
- US-A- 5 354 537
- US-A- 5 869 158
- US-A- 5 951 528

## Description

The invention relates to an elongated tubular needle cannula for injecting a fluid drug into a human body.

The invention furthermore relates to a method of producing such a needle cannula.

Finally the invention relates to the use of such needle cannula in a disposable syringe and in an injection needle assembly.

### Description of related art:

A catheter for a spinal anaesthesia procedure is disposed in US 5.002.535. This know catheter has an outside diameter at the skin piercing distal end which is smaller than the outside diameter at the opposite proximal end in order to provide a strong catheter which only makes a small hole in the dura mater when the catheter is removed thereby preventing leakage of cerebrospinal fluid from the spinal cord. The various diameters of the disclosed catheter is shown in the following table:

| | Skin piercing end | Opposite proximal end |
|---|---|---|
| Outside diameter (mm) | 0,45 - 0,63 | 0,63 - 1,25 |
| Inside diameter (mm) | 0,25 - 0,43 | 0,43 - 1,05 |

Due to the fact that the inside diameter of the skin piercing end is reduced compared to the inside diameter of the opposite end, the passage of fluid through the lumen is somewhat obstructed, and a relatively high pressure is necessary to force the fluid which can be feed into the large diameter at the opposite end through the reduced diameter of the skin piercing end.

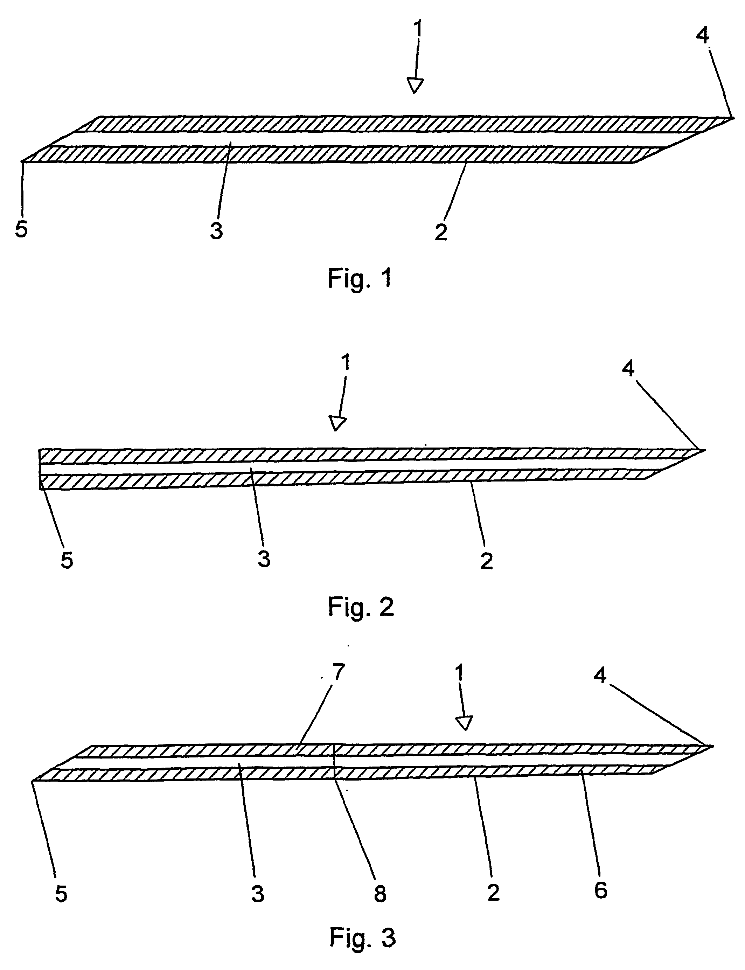

When injecting a drug into a human being, either as intramuscular injection or as subcutaneously injection, the injection needle used has a needle cannula with a substantially smaller diameter than the diameter of the disclosed catheter. Injection needles available today all have a needle cannula with both a uniform outside diameter and a uniform outside diameter, due to the fact that a needle cannula is drawn from a tube. Such a prior art needle cannula is illustrated in figure 1.

Some drugs, such as insulin are self-administered, and the typical diabetes person will require subcutaneous injections of insulin several times during the course of the day. Recent studies have indicated that people who inject themselves experience less pain when using a thin needle i.e. a needle cannula having a little outside diameter. In order to reduce the discomfort of having to inject oneself several times a day, injection needles with a very thin needle cannula are preferred among people suffering from diabetes.

The outside diameter of a needle cannula is indicated by a "G" followed by a gauge number, which gauge number increases with thinner needles. At the present, the most commonly used injection needles among people suffering from diabetes are G30 or G31. Thus the outside diameter of a G 30 is approximately 0,3 millimetres and of a G 31 approximately 0,26 millimetres

Injection needles for insulin delivery pens are disclosed in US 5.462.535. These known injection needles comprises a very thin G 30 needle cannula firmly fastened in a hub for removable mounting the injection needle onto, one of the many insulin delivery pens available today.

### Description of the Invention:

Injection needles having a very thin needle cannula do however present several problems.

Thin injection needles will undergo unintentional deflection when penetrating the tissue of the human body thereby building up a momentum at the junction where the needle cannula is fastened in the hub, which can lead to breakage of the needle cannula at the fastening point.

The injection rate of a thin needle cannula will be very slow, while known injection needles have a uniform inside diameter of the lumen, which decreases when the outside diameter decreases. Injection needles are usually made according to the ISO 9626 standard for Dimensions of tubing. According to this standard a G 30 needle has a minimum inside diameter of 0,133 millimetres whereas a G 31 needle cannula has a minimum inside diameter of only 0.114 millimetres, thus making the injection rate slower when decreasing the inside diameter of the lumen.

When decreasing. the inside diameter of the lumen, the. pressure needed to force the fluid drug through the lumen is increased. This again means that people injecting themselves has to press harder on the injection button on the insulin delivery device, which will cause excess pressure in the barrel or cartridge of the syringe.

Due to the reduced lumen of the thinner injection needles, clogging of insulin inside the lumen might occur.

It is henceforth an object of the present invention to provide a thin needle cannula having a reduced skin piercing end combined with a relatively large lumen, such that the pain perception is diminished without encountering the above mentioned flow problems.

This is obtained by a needle cannula according to claim 1.

### Explanation of claims 1 to 6:

The needle cannula of the present invention is usually made from metal, preferably steel and most preferably stainless steel, however a wide range of different alloys such as e.g. nickel-titanium could be used.

In the following the needle sizes G 31 and G 32 expresses only an example of needle sizes falling within the scope of the claims. The claimed invention is in no way limited to those specific sizes.

A G 31 needle cannula where the outside diameter of the skin piercing end is reduced to the diameter of a G 32 needle without reducing the inside diameter of the lumen provides a very attractive needle cannula. The reduction of the outside diameter is done in a way giving the needle cannula a conical tapered appearance. The skin piercing end penetrating the skin of the user is reduced in order to reduce the pain perception, while. the lumen is left with the inside diameter of a G 31 needle cannula. This provides the user of the needle cannula a perception of using a G 32 needle cannula, while the technical function of the needle cannula is that of a G 31 needle cannula.

Leaving the inside diameter of the needle cannula as a G 31 needle cannula allows an unobstructed passage of fluid through the lumen and prevents clogging in the lumen. Since the lumen has an inside diameter of a G 31 needle cannula, the user needs only apply an injection pressure substantially smaller than the injection pressure needed for injecting with a traditional G 32 needle cannula when injecting the fluid through the lumen.

At the opposite proximal end a G31 needle cannula according to the invention will have an outside diameter of a G 31 needle although the skin piercing end is reduced to the diameter of a G 32 needle cannula. This is very beneficial since the largest, and strongest, outside diameter will be at the fastening point, which is where the momentum is case of deflection of the needle cannula during injection is greatest.

Although the inside lumen of the needle cannula has approximately the same uniform inside diameter along the entire length of the needle cannula, the most distal end of the lumen can have a slightly tapered appearance due to the method of manufacturing as will be explained later.

In a preferred embodiment of the needle cannula according to the invention, the outside sidewall comprises two elongated tubular portions interfacing each other in a peripheral transition zone, namely a first elongated tubular portion extending from the peripheral transition zone to the skin piercing distal end of said needle cannula, and a second tubular portion extending from the peripheral transition zone to the opposite proximal end of said needle cannula.This divides the needle cannula into two parts, one part for entering the human body, and another part, which do not enter the human body.

The transition zone could in some cases be viewed as a transition point, but since the tapered appearance of the needle cannula is preferably made by dipping the needle cannula in a bath containing a metal eroding substance such as an acid as will be explained later, the area where the two elongated portions meets has more the configuration of a belt or zone than that of a point. The belt or zone is usually located in a distance of 1/4 to 2/3 of the length of the needle cannula inward from the skin piercing distal end.

Further, the first elongated tubular portion is conically tapered from the peripheral transition zone to the skin piercing distal end, and the second portion has approximately the same uniform outside diameter from the peripheral transition zone to the opposite proximal end of said cannula. This provides the first portion of the needle cannula inserted into a human body with the benefit of the reduced diameter, while the second portion of the needle cannula not entering the human body has a larger strength.

When, as disclosed in claim 3, the skin piercing distal end has an outside diameter equal to or smaller than a G 31 needle, the opposite. proximal end has an outside diameter equal to or larger than a G 30 needle, and the longitudinal lumen has an uniform inside diameter equal to or larger than the inside diameter of a G 30 needle, it is ensured that the needle. cannula operates as a G 30, or larger, needle cannula, while the pain perception is that of a G 31, or smaller, needle cannula.

When, as disclosed in claim 4, the skin piercing distal end has an outside diameter equal to or smaller than a G 32 needle, the opposite proximal end has an outside diameter equal to or larger than a G 31 needle, and the longitudinal lumen has an uniform inside diameter equal to or larger than the inside diameter of a G 31 needle, it is ensured that the needle cannula operates as a G 31, or larger, needle cannula, while the pain perception is that of a G 32, or smaller, needle cannula

When, as disclosed in claim 5 the difference in the outside diameter between the distal end and the proximal is 5 % or more, it is ensured that the difference approximately follows the steps defined in the ISO 9626 standard.

When, as disclosed in claim 6, both the skin piercing distal end and the opposite proximal end is sharpened, the needle cannula is especially suitable for an injection pen provided. with a cartridge containing the fluid to be injected, and where a barrier in the cartridge must be penetrated by the proximal end of the needle cannula in order to provide access to the fluid contained in the cartridge.

### Explanation of claims 7 to 8:

It is also the object of the present invention to provide a method of manufacturing a needle cannula having a reduced tip end. A method that must be both simple and economic to use in a large-scale production.

Such a method is obtained according to claim 7, and comprises the step of dipping at least a part of said needle cannula adjacent to and including the distal end into a metal eroding substance such as an acid containing bath for a controlled period of time, thereby removing a part of the needle cannula material.

By this method the material removed from the needle, cannula can be very exactly controlled, and the velocity by which the needle cannula is dipped into and pulled or hoisted out of the acid containing bath defines the shape of the conical part of the needle cannula. The needle cannula can of cause be dipped into the acid a number of subsequent times.

When, as disclosed in claim 8, the first elongated tubular portion extending. from the peripheral transition zone to the skin piercing distal end of said needle cannula is dipped in a bath containing a metal eroding substance, such as an acid for a controlled period of time leaving the second tubular portion extending from the peripheral transition zone to the opposite proximal end out of the metal eroding bath, a needle cannula suitable for use in a needle assembly for an injection pen is manufactured.

### Explanation of claim 9-11:

Finally it is the object of the present invention to provide a use of the needle cannula according to the invention either in the manufacture of a disposable syringe or an injection needle assembly. Such a use will be very attractive to people who has to inject them self several times every day, such as people suffering from diabetes.

This is obtained by the use according to claim 9 where the needle cannula is used in a disposable syringe comprising a barrel and plunger and wherein the needle cannula is permanently fastened to the barrel of the disposable syringe, or by the use according to claim 12 where the injection needle assembly comprises the needle cannula and a needle hub and wherein the needle cannula is permanently fastened in the needle hub.

When as disclosed in claim 11, the needle hub comprises a base and an annular sleeve extending from the base, the annular sleeve having means for removable mounting the hub onto a syringe, and the needle cannula being fastened in the base such that the first elongated tubular portion of the needle cannula extends from the base in a direction away from the sleeve, and the second tubular portion extends in the opposite direction and the second tubular portion is surrounded by the sleeve, it is ensured that the needle assembly is particular suitable for use on an injection pen.

### Definitions

Initially it may be convenient to define that the term "distal end" of the needle cannula according to invention is meant to refer to the end, which is forced to penetrate the skin of the human body when injecting a fluid, whereas the term "proximal end" is meant to refer to the opposite end of the needle cannula which in use points away from the human body.

It is to be understood that the wording "outside diameter at the skin piercing distal end", refers to the outside diameter of the most distal end. of the needle cannula. This most distal end is however often cut in an oblique shape to facilitate the penetration of the skin of the human body, which makes its difficult exactly to measure the outside diameter at the most distal end. It is therefore sometimes necessary to measure the diameter right before the oblique cut, which diameter then falls within the definition of the diameter of the distal end. The same is the case if the opposite proximal end has an oblique cut, then the outside diameter of the opposite proximal end could be the diameter adjacent the oblique cut.

Although the wording "human body" is used throughout this application, the needle cannula could as well be used on any mammal body without dispersing from the scope of the claims.

It is to be understood that the. wording "injection pen", merely refers to an injection device having an oblong or elongated shape, somewhat like a pen for writing. Although such pens usually have a tubular cross-section, modem writing pens often have a different cross-section such as triangular, rectangular or square. A pen shaped housing can in a similar way have a large variety of different cross-sections.

G31 to G33 is in the ISO 9626 standard defined as:

| Gauge size | G31 | G32 | G33 |
|---|---|---|---|
| Designated metric size | 0,25 mm | 0,23 mm | 0,20 mm |
| Minimum outside diameter | 0,254 mm | 0,229 mm | 0,203 mm |
| Maximum outside diameter | 0,267 mm | 0,241 mm | 0,216 mm |
| Minimum inside diameter | 0,114 mm | 0,089 mm | 0,089 mm |

Although the referred ISO standard does not cover tapered tubing as such, the gauge dimensions from the ISO standard is used throughout this application merely to indicate the dimensions at specific locations.

### Brief Description of the Drawings:

The invention will be explained more fully below in connection with a preferred embodiment and with reference to the drawings in which:
- Figure 1: Shows a sectional side view of a prior art needle cannula.
- Figure 2: Shows a sectional view of a needle cannula according the invention.
- Figure 3: Shows a sectional view of a needle cannula according to an embodiment of the invention.
- Figure 4: Shows a view of a needle cannula according to the invention mounted on a disposable syringe.
- Figure 5: Shows a view of a needle cannula according to the invention mounted in a hub.
- Figure 6: Shows a view of a needle cannula according to an embodiment of the invention mounted in a hub.
- Figure 7: Shows a view of a needle cannula according to the invention dipped in an acid containing bath.

The figures are schematic and simplified for clarity, and they just show details, which are essential to the understanding of the invention, while other details are left out. Throughout, the same reference numerals are used for identical or corresponding parts.

### Detailed Description of Embodiments:

The needle cannula 1 according to the present invention can be utilized either for injecting a fluid into the body, or for retracting a fluid from the body.

Figure 1 show a needle cannula 1 made up from an elongated tube, which is drawn until the. desired diameter is obtained. Both the outside diameter of the. needle cannula 1 and the inside diameter of the needle cannula 1 are approximately uniform throughout the entire length of the tube making up the needle cannula 1. The inside cylindrical and longitudinal lumen 3 of the needle cannula 1 is therefore parallel with the outside surface 2 of the needle cannula 1.

The needle cannula 1 has a distal end 4 and a proximal end 5. The distal end 4 is sharpened for piercing the skin of the human being injected. The proximal end 5 is connected to a not shown fluid delivery apparatus, delivering fluid into a human being through the lumen 3 of the needle cannula 1.

The diameter of the lumen 3 must be chosen to accommodate the fluid to be injected, such that the particular fluid can flow through the lumen 3 without clogging. When injecting insulin the minimum diameter of the lumen 3 of the needle cannula 1 is today generally considered as being that of a G 31 needle e.g. 0,114 mm, hence making the outside diameter approximately 0,26 mm.

As shown in figure 2 the outside surface 2 of the needle cannula 1 can be made to taper towards the distal end 4, such that the outside diameter of the distal 4 end is smaller than the outside diameter of the proximal end 5. At the same time the inside cylindrical and longitudinal lumen 3 continues to have a uniform diameter.

The tapered shape, which is usually conical, can be obtained either by forging the needle cannula e.g. with a mandrel mounted in the. lumen 3 or by removing a part of the material making up the needle cannula 1. The material can be removed by grinding a part of the material of the needle cannula away, or by applying a metal eroding substance such as an acid to the needle cannula 1, which e.g. could be done by dipping the needle. cannula 1 into an acid containing bath for a controlled period of time.

The inside diameter of the tapered needle cannula 1 is uniform and equal to the inside diameter of the original needle cannula 1 throughout the entire length. If a G 31 needle cannula 1 is used the inside diameter is 0,114 mm while the outside diameter at the proximal end is approximately 0,26 mm. The outside diameter of the distal end is then reduced to that of a G 32 needle i.e. approximately 0,23 mm, thereby lowering the pain perception of the needle cannula but keeping the same flow through the lumen 3 of the needle cannula 1.

The following table indicates the outside diameter of the proximal end 5 and of the skin piercing distal end 4 of a needle cannula 1 according to the present invention as they appear in the ISO 9626 standard. The minimum inside diameter of the lumen 3 is also indicated in the table. The tolerances for these numbers is usually +/- 0,01 mm on the actual outside diameter.

| Conical gauges | G30 ― G31 | G31 ― G32 | G32 ― G33 |
|---|---|---|---|
| Diameter, proximal end | 0,298 - 0,320 mm | 0,254 - 0,267 mm | 0,229 - 0,241 mm |
| Diameter, distal end | 0,254 - 0,267 mm | 0,229 - 0,241 mm | 0,203 - 0,216 mm |
| Min Diam., inside lumen | 0,133 mm | 0,114 mm | 0,089 mm |

As shown on figure 3 the needle cannula needs not to be conical tapered along the entire length. Instead the outside sidewall of the. needle cannula 1 can be divided into two elongated tubular portions 6, 7 interfacing each other in a peripheral transitions zone. 8. The first part 6 extends from the distal skin-piercing end 4 to the peripheral transition zone 8, while the second part 7 extends from the proximal end to the peripheral transitions zone 8. The first part 6 is conically tapered from the peripheral transitions zone 8 towards the skin piercing distal end 4, while the second part 7 has approximately the same uniform diameter from the peripheral transitions zone 8 to the proximal end 5. The inside diameter of the longitudinal lumen 3 is uniform along the entire length of the needle cannula.

The outside diameter of the second part 7 could be that of a G 31 needle, while the outside diameter at the distal end 4 could be that of a G 32 needle. The inside diameter which is uniform along the entire length could be equal to the diameter of a G 31 needle. This would provide the user with a feeling of injecting with a G 32 needle while the flow through the lumen of the needle cannula 1 equals that of a G 31 needle.

It must however be stressed, that the exact diameters of the needle cannula 1 does not necessarily have to follow the ISO standard as long as the claimed principle of having a reduced diameter at the skin piercing end 4 combined with a cylindrical lumen 3 is followed.

In practical use the needle cannula 1 of the invention is mounted either on a disposable syringe 9 or in a needle hub 12,13.

The needle cannula 1 shown in figure 2 and figure 3 is preferably manufactured by dipping the distal end of the needle cannula 1 into an add containing bath, as will be described later. Alternatively the tapered second part 6 could med manufactured by it self and welded on to the first part 7.

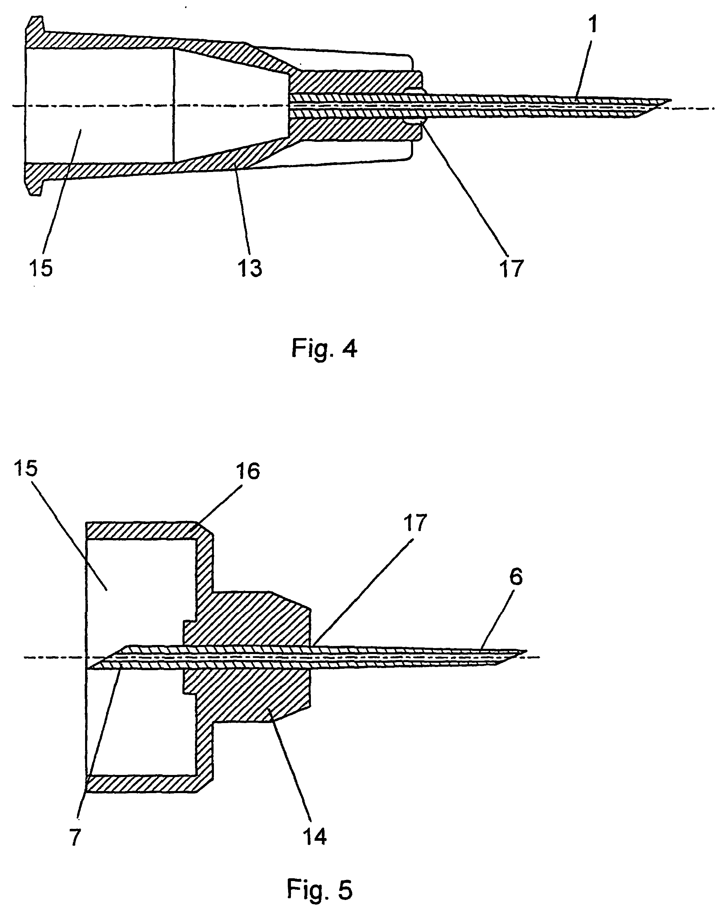

Figure 4 and 5 shows the needle cannula 1 permanently fastened in a needle hub 13,14, which needle hub 13,14 have means for removable mounting the needle hub 12 onto a syringe.

Figure 4 show a traditional needle assembly for intramuscular injection of a fluid. The needle cannula 1 is fastened in the needle hub 13 such that the proximal end 5 of the needle cannula 1 connects to the cylindrical opening 15 in the needle hub 13 into which opening 15 the tip of a not shown hypodermic syringe is pushed when mounting the needle hub 1 onto a hypodermic syringe.

The needle cannula 1 shown in figure 2, which is tapered along the entire length of the needle cannula 1 is particular suitable for this type of needle assembly.

Figure 5 shows a needle assembly for use on a pen system where an injection pen is provided with a cartridge containing the fluid to be injected, and where a barrier in the cartridge must be penetrated in order to provide access to the fluid contained in the cartridge. The needle cannula 1 of the needle assembly is divided into a first part 6, which penetrates into the skin of a human being, and a second part 7, which penetrates into the cartridge containing the fluid, when the needle assembly is mounted on the injection pen. The skirt 16 surrounding the cylindrical opening 15 into which opening 15 the injection pen is inserted, usually carries means, such as a thread, for holding the needle assembly on to the injection pen.

The needle cannula 1 shown in figure. 3 is particular suitable for this type of needle assembly, since the second part 7 of the needle cannula 1 has a uniform diameter larger than the diameter of the first part 6. This provides a second part 7, which is more reluctant to bending than the first part 6. This is to be preferred since the second part of 7 the needle cannula 1 has to penetrate the barrier of the cartridge.

No matter which of the needle hubs 13, 14 are used, the needle cannula 1 must be located in the needle hub 13,14 such that the needle cannula 1 has a relatively large diameter at the junction 17 between the needle cannula 1 and the needle hub 13, 14. When bending the needle cannula 1 during injection, the largest torque will be exerted right at this junction 17. It is therefore important that the needle cannula 1 has a relatively large diameter and thereby a large resistant against bending at the junction 17. This could e.g. be provided if the peripheral transition zone 8 of the needle, cannula 1 shown in figure. 3 is located right at the junction 17 between the needle cannula 1 and the needle hub 14.

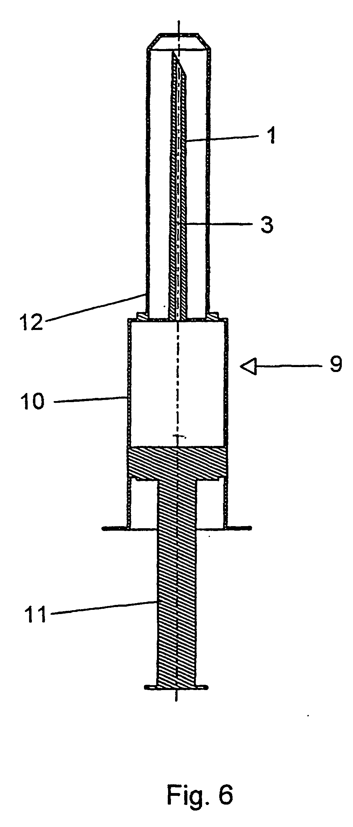

Figure 6. shows the needle cannula 1 permanently fastened to a disposable syringe 9. The disposable syringe 9 comprises a barrel 10 containing the fluid to be injected and a plunger 11, which is moved forward in order to press the fluid trough the lumen 3 of the needle cannula 1. A removable needle cover 12 can cover the needle cannula 1 when the syringe 9 is not in use.

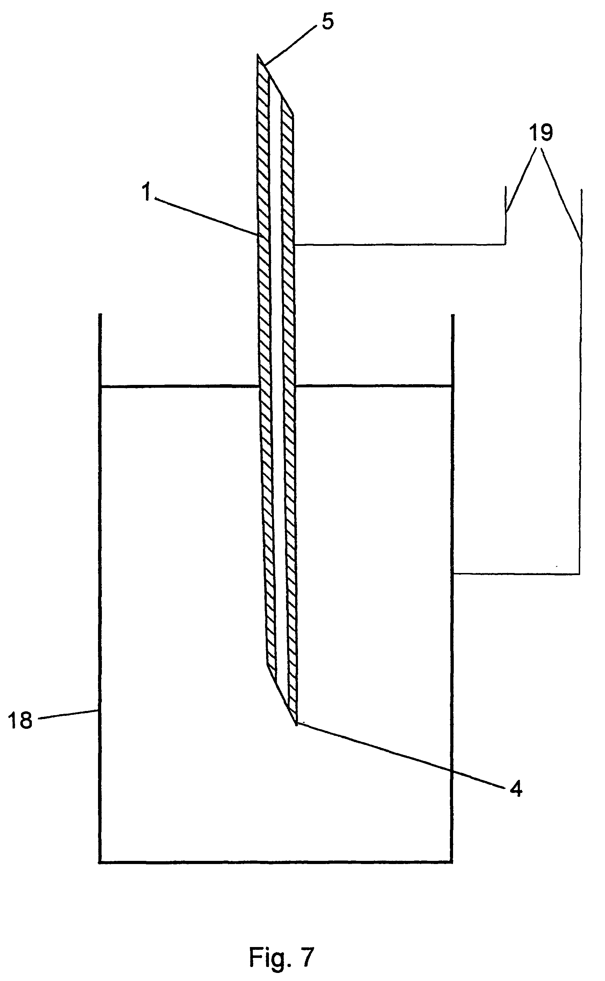

A preferred method of manufacturing a metallic needle cannula according to the invention is shown in figure. 7. The part of the metallic needle cannula 1 which diameter is intended to be reduced is dipped in a bath containing a metal eroding substance such as an acid. In order to increase the removal of material from the needle cannula 1 electrical wires 19 can apply a current between the needle cannula 1 and the container 18 containing the acid, or a not shown cathode which is dipped in the bath, such that the needle cannula 1 works as the anode of an electrolytic process.

The needle. cannula 1 can either be. dipped in the. acid containing bath one time or a number of subsequent times. Experiments has demonstrated. that lowering a stainless steel needle cannula into a bath containing a 74% phosphoric acid for approximately 80 times each of 1,5 seconds, a total of 120 seconds, provides a very attractive result.

When the needle cannula 1 is dipped in the acid containing bath, some of the acid can flow into the lumen 3 of the needle cannula 1 and remove some of the material on the inside surface of the distal end 4 of needle cannula 1, making the distal part of the lumen 3 to taper towards the proximal end 5 of the needle cannula 1. For some needle applications this is to be preferred, but could however be prevented by applying a pressure to the lumen 3 while dipping the needle cannula 1 in the acid containing bath. This could e.g. be done by blowing gas into the lumen through the proximal end 5 of the needle cannula 1 while dipping the distal end 4 of the needle cannula 1 in the acid-containing bath. In this way acid can be effectively prevented from entering the lumen 3 of the needle cannula 1.

Some preferred embodiments have been shown in the foregoing, but it should be stressed that the invention is not limited to these, but may be embodied in other ways within the subject matter defined in the following claims.

## Claims

1. An elongated tubular needle cannula (1) for injecting a fluid into a human body or retracting a fluid from the human body, comprising;
a skin piercing distal end (4), an opposite proximal end (5) and a side wall (2) there between, having a conical tapered outside, at least at the skin piercing distal end (4),
a lumen (3) extending through said needle cannula (1) from the skin piercing distal end (4) to the opposite proximal end (5) along a longitudinal axis of said needle cannula (1), said needle cannula (1) having an outside diameter at the skin piercing distal end (4) which is smaller than the outside diameter at the opposite proximal end (5),
**Characterized in that**,
the longitudinal lumen (3) is cylindrical and has approximately the same uniform inside diameter through said needle cannula (1) from the skin piercing distal end (4) to the opposite proximal end (5).

2. An elongated tubular needle cannula according to claim 1, **characterized in that**, the outside sidewall (2) comprises two elongated tubular portions (6, 7) interfacing each other in a peripheral transition zone (8), namely a first elongated tubular portion (6) extending from the peripheral transition zone (8) to the skin piercing distal end (4) of said needle cannula (1), and a second tubular portion (7) extending from the peripheral transition zone (8) to the opposite proximal end (5) of said needle cannula (1), the first elongated tubular portion (6) being conically tapered from the peripheral transition zone (8) to the skin piercing distal end (4), and the second portion (7) having approximately the same uniform outside diameter from the peripheral transition zone (8) to the opposite proximal end (5) of said cannula (1).

3. An elongated tubular needle cannula according to claim 1 or 2 , **characterized in that** the skin piercing distal end (4) has an outside diameter equal to or smaller than a G 31 needle, the opposite proximal end (5) has an outside diameter equal to or larger than a G 30 needle, and the longitudinal lumen (3) has an uniform inside diameter equal to or larger than the inside diameter of a G 30 needle.

4. An elongated tubular needle cannula according to claim 1 or 2 , **characterized in that** the skin piercing distal end (4) has an outside diameter equal to or smaller than a G 32 needle, the opposite proximal end (5) has an outside diameter equal to or larger than a G 31 needle, and the longitudinal lumen (3) has an uniform inside diameter equal to or larger than the inside diameter of a G 31 needle.

5. An elongated tubular needle cannula according to anyone of the preceding claims, **characterized in that** the distal end (4) is approximately 5 % or more small in outside diameter than the proximal end (5).

6. An elongated tubular needle cannula according to anyone of the preceding claims, **characterized in that** both the skin piercing distal end (4) and the opposite proximal end (5) is sharpened.

7. A method of manufacturing a needle cannula according to claim 1 to 6 , **characterized in that**, the method comprises the step of dipping the needle cannula (1) or a part of said needle cannula (1) adjacent to and including the distal end (4) into a metal eroding substance such as an acid containing bath (18) for a controlled period of time, thereby removing a part of the needle cannula material.

8. A method of manufacturing a needle cannula according to claim 7 **characterized in that** the first elongated tubular portion (6) extending from the peripheral transition zone (8) to the skin piercing distal end (4) of said needle cannula (1) is dipped in the metal eroding bath (18) for a controlled time leaving the second tubular portion (7) extending from the peripheral. transition zone (8) to the opposite proximal end (5) out of the metal eroding bath (18)

9. Use of the needle cannula of claim 1 to 6 in the manufacture of a disposable syringe comprising a barrel (10) and plunger (11) and wherein said needle, cannula (1) is permanently fastened to the barrel (10) of said disposable syringe (9).

10. Use of the needle cannula of claim 1 to 6 in the manufacture of an injection needle assembly comprising said needle. cannula (1) and a needle hub (13, 14) and wherein said needle cannula (1) is permanently fastened in said needle hub (13, 14).

11. The use according to claim 10, **characterized in that**, said needle hub (14) comprises a base and an annular sleeve (16) extending from the base, said annular sleeve (16) having means for removable mounting said hub (14) onto a syringe, and said needle cannula (1) being fastened in the base such that the first elongated tubular portion (6) of said needle cannula (1) extends from the base in a direction away from the sleeve (16), and the second tubular portion (7) extends in the opposite direction, said second tubular portion (7) being at least partly surrounded by the sleeve (16).

## Patentansprüche

1. Längliche, röhrenförmige Nadelkanüle (1) zum Injizieren einer Flüssigkeit in einen menschlichen Körper oder Einziehen einer Flüssigkeit aus einem menschlichen Körper, umfassend:
ein die Haut durchstechendes distales Ende (4), ein gegenüberliegendes proximales Ende (5) und dazwischen eine Seitenwand (2) mit einer konisch zulaufenden Außenseite zumindest am die Haut durchstechenden distalen Ende,
ein Lumen (3), das sich durch die Nadelkanüle (1) vom die Haut durchstechenden Ende (4) zu dem gegenüberliegenden proximalen Ende (5) entlang einer Längsachse der Nadelkanüle (1) erstreckt, wobei die Nadelkanüle (1) einen Außendurchmesser am die Haut durchstechenden distalen Ende (4) aufweist, der kleiner als der Außendurchmesser am gegenüberliegenden proximalen Ende ist,
**dadurch gekennzeichnet, dass**
das längliche Lumen (3) zylinderförmig ist und in etwa denselben gleichförmigen Innendurchmesser durch die Nadelkanüle (1) von dem die Haut durchstechenden distalen Ende (4) zu dem gegenüberliegenden proximalen Ende (5) aufweist.

2. Längliche, röhrenförmige Nadelkanüle nach Anspruch 1, **dadurch gekennzeichnet, dass** die Außenseitenwand (2) zwei längliche, röhrenförmige Teile (6, 7), die miteinander in einer peripheren Übergangszone (8) verbunden sind, d. h. ein erstes längliches, röhrenförmiges Teil (6), das sich von der peripheren Übergangszone (8) zu dem die Haut durchstechenden distalen Ende (4) der Nadelkanüle (1) erstreckt, und ein zweites röhrenförmiges Teil (7), das sich von der peripheren Übergangszone (8) zu dem gegenüberliegenden proximalen Ende (5) der Nadelkanüle (1) erstreckt, umfasst, wobei das erste längliche, röhrenförmige Teil (6) von der peripheren Übergangszone (8) zu dem die Haut durchstechenden distalen Ende (4) konisch zuläuft und das zweite Teil (7) etwa denselben gleichförmigen Außendurchmesser von der peripheren Übergangszone (8) zu dem gegenüberliegenden proximalen Ende (5) der Kanüle (1) aufweist.

3. Längliche, röhrenförmige Nadelkanüle nach Anspruch 1 oder 2, **dadurch gekennzeichnet, dass** das die Haut durchstechende distale Ende (4) einen Außendurchmesser aufweist, der gleich oder kleiner als eine G31-Nadel ist, das gegenüberliegende proximale Ende (5) einen Außendurchmesser aufweist, der gleich oder größer als eine G30-Nadel ist, und das längliche Lumen (3) einen gleichförmigen Innendurchmesser aufweist, der gleich oder größer als der Innendurchmesser einer G30-Nadel ist.

4. Längliche, röhrenförmige Nadelkanüle nach Anspruch 1 oder 2, **dadurch gekennzeichnet, dass** das die Haut durchstechende distale Ende (4) einen Außendurchmesser aufweist, der gleich oder kleiner als eine G32-Nadel ist, das gegenüberliegende proximale Ende (5) einen Außendurchmesser aufweist, der gleich oder größer als eine G31-Nadel ist, und das längliche Lumen (3) einen gleichförmigen Innendurchmesser aufweist, der gleich oder größer als der Innendurchmesser einer G31-Nadel ist.

5. Längliche, röhrenförmige Nadelkanüle nach einem der vorangehenden Ansprüche, **dadurch gekennzeichnet, dass** das distale Ende (4) im Außendurchmesser um etwa 5% oder mehr kleiner als das proximale Ende (5) ist.

6. Längliche, röhrenförmige Nadelkanüle nach einem der vorangehenden Ansprüche, **dadurch gekennzeichnet, dass** sowohl das die Haut durchstechende distale Ende (4) als auch das gegenüberliegende proximale Ende (5) angespitzt ist.

7. Verfahren zur Herstellung einer Nadelkanüle nach Anspruch 1 bis 6, **dadurch gekennzeichnet, dass** das Verfahren den Schritt des Eintauchens der Nadelkanüle (1) oder eines Teils der Nadelkanüle (1) nahe am und einschließend das distale(n) Ende (4) in eine metallätzende Substanz wie ein säurehaltiges Bad (18) über eine kontrollierte Zeitdauer, um damit einen Teil des Nadelkanülenmaterials zu entfernen.

8. Verfahren zur Herstellung einer Nadelkanüle nach Anspruch 7, **dadurch gekennzeichnet, dass** das erste längliche, röhrenförmige Teil (6), das sich von peripheren Übergangszone (8) zu dem die Haut durchstechenden distalen Ende (4) der Nadelkanüle (1) erstreckt, in das metallätzende Bad (18) über eine kontrollierte Zeitdauer getaucht wird, indem das zweite röhrenförmige Teil (7), das sich von der peripheren Übergangszone (8) zu dem gegenüberliegenden proximalen Ende (5) erstreckt, außerhalb des metallätzenden Bads gelassen wird.

9. Verwendung der Nadelkanüle nach Anspruch 1 bis 6 bei der Herstellung einer einen Zylinder (10) und einen Kolben (11) umfassenden Wegwerfspritze, und wobei die Nadelkanüle (1) dauerhaft an dem Zylinder (10) der Wegwerfspritze (9) befestigt ist.

10. Verwendung der Nadelkanüle nach Anspruch 1 bis 6 bei der Herstellung eines Injektionsnadelgefüges, umfassend die Nadelkanüle (1) und eine Nadelnabe (13, 14), und wobei die Nadelkanüle (1) dauerhaft in der Nadelnabe (13, 14) befestigt ist.

11. Verwendung nach Anspruch 10, **dadurch gekennzeichnet, dass** die Nadelnabe (13, 14) eine Basis und eine sich von der Basis erstreckende, ringförmige Hülse (16) umfasst, wobei die ringförmige Hülse (16) Elemente zum entfernbaren Anbringen der Nabe (14) auf einer Spritze aufweist und die Nadelkanüle (1) in der Basis so befestigt ist, dass sich das erste längliche, röhrenförmige Teil (5) der Nadelkanüle (1) von der Basis in einer Richtung weg von der Hülse (16) und das zweite röhrenförmige Teil (7) sich in Gegenrichtung erstreckt, wobei das zweite röhrenförmige Teil (7) zumindest teilweise von der Hülse (16) umgeben ist.

## Revendications

1. Canule aiguille tubulaire allongée (1) pour injecter un fluide dans un corps humain ou extraire un fluide du corps humain, comprenant :
une extrémité distale perçant la peau (4), une extrémité proximale opposée (5) et une paroi latérale (2) entre elles, ayant un extérieur conique progressivement rétréci, au moins au niveau de l'extrémité distale perçant la peau (4) ;
un lumen (3) s'étendant à travers ladite canule aiguille (1) à partir de l'extrémité distale perçant la peau (4) vers l'extrémité proximale opposée (5) le long d'un axe longitudinal de ladite canule aiguille (1), ladite canule aiguille (1) ayant un diamètre externe au niveau de l'extrémité distale perçant la peau (4) qui est inférieur au diamètre externe au niveau de l'extrémité proximale opposée (5),
**caractérisée en ce que** le lumen longitudinal (3) est cylindrique et a approximativement le même diamètre intérieur uniforme à travers ladite canule aiguille (1) de l'extrémité distale perçant la peau (4) à l'extrémité proximale opposée (5).

2. Canule aiguille tubulaire allongée selon la revendication 1, **caractérisée en ce que** la paroi latérale externe (2) comprend deux parties tubulaires allongées (6, 7) s'interfaçant entre elles dans une zone de transition périphérique (8), c'est-à-dire une première partie tubulaire allongée (6) s'étendant de la zone de transition périphérique (8) à l'extrémité distale perçant la peau (4) de ladite canule aiguille (1), et une seconde partie tubulaire (7) s'étendant de la zone de transition périphérique (8) à l'extrémité proximale opposée (5) de ladite canule aiguille (1), la première partie tubulaire allongée (6) étant rétrécie progressivement de manière conique de la zone de transition périphérique (8) à l'extrémité distale perçant la peau (4), et la seconde partie (7) ayant approximativement le même diamètre externe uniforme de la zone de transition périphérique (8) à l'extrémité proximale opposée (5) de ladite canule aiguille (1).

3. Canule aiguille tubulaire allongée selon la revendication 1 ou 2, **caractérisé en ce que** l'extrémité distale perçant la peau (4) a un diamètre externe égal à ou inférieur à une aiguille G31, l'extrémité proximale opposée (5) a un diamètre externe égal à ou supérieur à une aiguille G30, et le lumen longitudinal (3) a un diamètre interne uniforme égal à ou supérieur au diamètre interne d'une aiguille G30.

4. Canule aiguille tubulaire allongée selon la revendication 1 ou 2, **caractérisée en ce que** l'extrémité distale perçant la peau (4) a un diamètre externe égal à ou inférieur à une aiguille G32, l'extrémité proximale opposée (5) a un diamètre externe égal à ou supérieur à une aiguille G31, et le lumen longitudinal (3) a un diamètre interne uniforme égal à ou supérieur au diamètre interne d'une aiguille G31.

5. Canule aiguille tubulaire allongée selon l'une quelconque des revendications précédentes, **caractérisée en ce que** l'extrémité distale (4) a un diamètre externe représentant environ 5% ou moins de l'extrémité proximale (5).

6. Canule aiguille tubulaire allongée selon l'une quelconque des revendications précédentes, **caractérisée en ce qu'**à la fois l'extrémité distale perçant la peau (4) et l'extrémité proximale opposée (5) est affûtée.

7. Procédé de fabrication d'une canule aiguille selon les revendications 1 à 6, **caractérisée en ce que** le procédé comprend l'étape consistant à immerger la canule aiguille (1) ou une partie de ladite canule aiguille (1) adjacente à, et comprenant, l'extrémité distale (4) dans une substance d'érosion de métal tel qu'un bain contenant de l'acide (18) pendant une période de temps contrôlée, retirant ainsi une partie du matériau de la canule aiguille.

8. Procédé de fabrication d'une canule aiguille selon la revendication 7, **caractérisé en ce que** la première partie tubulaire allongée (6) s'étendant de la zone de transition périphérique (8) à l'extrémité distale perçant la peau (4) de ladite canule aiguille (1) est immergée dans le bain d'érosion de métal (18) pendant un temps contrôlé laissant la seconde partie tubulaire (7) s'étendant de la zone de transition périphérique (8) à l'extrémité proximale opposée (5) hors du bain d'érosion de métal (18).

9. Utilisation de la canule aiguille selon les revendications 1 à 6 dans la fabrication d'une seringue jetable comprenant un cylindre (10) et un piston (11) et dans laquelle ladite canule aiguille (1) est fixée de manière permanente au cylindre (10) de ladite seringue jetable (9).

10. Utilisation de la canule aiguille selon les revendications 1 à 6 dans la fabrication d'un ensemble d'aiguille à injection comprenant ladite canule aiguille (1) et un raccord d'aiguille (13, 14) et dans laquelle ladite canule aiguille (1) est fixée de manière permanente audit raccord d'aiguille (13, 14).

11. Utilisation selon la revendication 10, **caractérisée en ce que** ledit raccord d'aiguille (14) comprend une base et un manchon annulaire (16) s'étendant à partir de la base, ledit manchon annulaire (16) possédant des moyens pour le montage amovible dudit raccord (14) sur une seringue, et ladite canule aiguille (1) étant fixée dans la base de sorte que la première partie tubulaire allongée (6) de ladite canule aiguille (1) s'étend à partir de la base dans une direction à distance du manchon (16), et la seconde partie tubulaire (7) s'étend dans la direction opposée, ladite seconde partie tubulaire (7) étant au moins partiellement entourée par le manchon (16).
